# EUROPEAN PATENT APPLICATION

(11) **EP 1 287 828 A1**
(43) Date of publication of application: **05.03.2003**
(21) Application number: 01119755.5
(22) Date of filing: 28.08.2001
(51) Int. Cl.: A61K 35/78, A23L 1/30

(54) **Composition of health food for prophylaxis and treatment of constipation**

(71) Applicant: Han, Wan-Seok, 135-894 Seoul (KR)
(72) Inventor: Wan-Seok Han Whayang Bldg., 2nd floor, 135-894 Seoul (KR)
(74) Representative: Müller, Frank Peter, Dipl.-Ing.

(57) **Abstract**

Disclosed is a composition for use in health foods for the prophylaxis and treatment of constipation, prepared from medicinal herbs, including Angelica gigas Nakai, Astragalus membranaceus (Fisch.) Bunge, Paeonia aliflora Pallas var. trichocarpa Bunge, Atractylodes japonica (Koidz.) Kitag, Crataegus pinnatifida Bunge, Zanthoxylum piperitum (L.) DC., Lycium chinense Miller, Macrocarpium officinale Sieb. et Zucc., and Glycyrrhiza glabra L. In addition to being safe to the body, the composition exhibits excellent pharmaceutical effects and provide contentment for the user without side effects.

## Description

### 1. Field of the invention

The present invention relates, in general, to a composition of health foods for the prophylaxis and treatment of constipation and, more particularly, to a composition made of medicinal herbs, including *Angelica gigas Nakai*, Astragalus *membranaceus* (*Fisch*.) *Bunge*, *Paeonia aliflora Pallas var*. *trichocarpa Bunge, Atractylodes japonica (Koidz.) Kitag*, *Crataegus pinnatifida Bunge*, *Zanthoxylum piperitum (L.) DC*., *Lycium chinense Miller, Macrocarpium officinale Sieb. et Zucc.,* and *Glycyrrhiza glabra L.,* suitable for use in health foods for the prophylaxis and treatment of constipation.

### 2. Description of the Prior Art

These days, high-calorie diets such as high-fat diets and fast foods are popularized in developed countries. Generally, those who eat such high-calorie diets, but do not ingest fibrous materials nor take exercise are liable to suffer from obesity and constipation. In fact, the number of patients suffering form obesity and constipation is on upward tend. Now, obesity and constipation are recognized as serious disorders threatening the health management of modem people.

Constipation may be defined as infrequent or hard stools or difficulty passing stools. Constipation is a relative term. There is wide variability is what is considered normal patterns of bowel evaculation. While some healthy people may have consistently soft or near runny stools others may have consistently hard firm stools, but no difficulty in passing them. When the stool is hard, infrequent, and requires significant effort to pass, the person has constipation. Quantitatively, the person who has 30 g or less of rigid excrements daily or evacuates the bowels twice or less times a week is diagnosed to have constipation. Constipation may cause discomfort with passage of stools, and passage of large stools may tear the mucosal membrane of the anus, especially in children, causing bleeding and the possibility of an anal fissure. Once afflicted with constipation, persons may suffer from complications such as headache or skin rash, in serious cases, piles, and in the worst case, rectal cancer. Constipation can be caused by changes in diet, decrease in physical activity, lack of toilet facilities, behavior and psychological problems, dehydration, diseases of the bowel, neurological diseases, congenital diseases, medications, and many other causes.

Constipation means the residing of excrements in the colon for a longer time period than usual. As a rule, foods are excreted as dung 12 to 72 hours after their ingestion. Kinds of ingested foods have considerable influence on the excretion time. For instance, fiber-rich foods increase in volume by absorbing water in the intestines so as to promote the passing of stools. However, low-fiber diets do not promote evacuation.

In order to prevent constipation, regular diets are necessary. Occasional fasting may disturb habitual bowel movements. Chronic constipation may result from physiological difficulty in passing stools or from physical troubles associated with diseases. Alternatively, chronic constipation may be caused by senile dysfunction. In general, there are basically three types of constipation; and this classification relates back to the causes of the ailment.

Flaccid or atonic constipation is the most common and is the result of a lack of sufficient fluids in the diet. This ailment results from insufficient muscle contraction or low activity. In other words, the activity of the intestines to perform excretion is insufficient, due to weak peristalsis, so that the intestinal contents move slowly. This type of constipation often occurs in the elderly whose metabolic activity is lowered. Also, this aliment is found in those who suffer from obesity or fever, undergo surgical operation, or are pregnant. It occurs due mainly to a lack of roughage in diet, irregular meal hours, fasting, insufficient time period to pass stools, and not taking sufficient water or adequate fruits and vegetables (and hence lack of bulk-forming cellulose).

The second type is convulsive or spastic constipation, a kind of an excitatory colopathy, having causes opposite from those of the flaccid constipation. Convulsive or spastic constipation is caused by the excessive contraction of the nerve endings in the intestines. In this case, the sigmoid colon is of excessive activity or its action is not harmonious with that of other sections of the colon. In addition, the strength of the rectal contraction tends to decrease. Persons who are afflicted with this type of constipation often suffer from abdominal pain and feel nausea. Also, they have loose bowels alternating with constipation with concomitant excretion of mucus. Causes relevant to convulsive or spastic constipation are diverse, including very coarse diets, drinking too much tea and coffee and alcohol, the frequent use of purgatives, heavy smoking, intestinal infection, tension, emotional turmoil, and environmental factors (insufficient sleep, rest, and water ingestion). The patients feel unpleasant and suffer intestinal expansion and abdominal pains and may be seriously convulsed with the abdomen bulging. They are of underweight and of nervous temperament. Also, they are afraid of eating owing to the memory of previous pain and seized with fear of future pain.

The third type - obstructive constipation - is usually due to the malignancy of the colon, what is known as the 'impacted colon' and may require surgery. Symptoms of severely constipated people include heaviness of head, insomnia, a furred, coated tongue, foul breath, headache or dizziness, and a loss of appetite.

For the treatment of constipation, its causes, if they are identified, such as metabolism or endocrine disturbance, nervous tissue dysfunction, anal diseases, etc., must be treated in advance. However, patients with functional constipation, whose causes are not identifiable, are primarily required to change their lifestyles and undergo bowel training. Particularly, it is recommended for the patient to cultivate the habit of passing stools after breakfast. Additionally, a dietary treatment is necessary, together with the bowel training. Ingestion of fibrous materials and water not only improves the transportation of foods along digestive tracts, but also absorbs sloughed intestinal cells and mucous to increase the amount of stools, thereby making excretion easy. For these reasons, functional foods for treating constipation have been under extensive study.

### SUMMARY OF THE INVENTION

Leading to the present invention, the intensive and thorough research to develop health foods for promoting the circulation of blood, conducted by the present inventor aiming to overcome the problems encountered in prior arts, resulted in the finding that certain wild herbs promote the functions of the organs relevant to excretion and are medicinally effective for the prophylaxis and treatment of constipation.

Therefore, it is an object of the present invention to provide a composition of health foods for the prophylaxis and treatment of constipation, which is safe to the body and can allow the user to have regular and easy bowel movements.

In accordance with the present invention, the above object could be accomplished by a provision of a composition of health foods for the prophylaxis and treatment of constipation, comprising: 8-15 % by weight of *Angelica gigas Nakai*, 8-15 % by weight of *Astragalus membranaceus (Fisch.) Bunge,* 8-15 % by weight of *Paeonia aliflora Pallas var*. *trichocarpa Bunge,* 8-15 % by weight of *Atractylodes japonica (Koidz.) Kitag*, 8-15 % by weight of *Crataegus pinnatifida Bunge,* 8-15 % by weight of *Zanthoxylum piperitum (L.) DC.,* 8-15 % by weight of *Lycium* chinense *Miller,* 8-15 % by weight of *Macrocarpium officinale Sieb*. *et Zucc.,* and 2-20 % by weight of *Glycyrrhiza glabra L.*

### DETAILED DESCRIPTION OF THE INVENTION

*Angelica gigas Nakai* is a perennial herb which grows to a height of 1-2 m. It grows at damp sites in mountain valleys that rise 200-1,500 m above the sea level. Roots of female Angelica gigas Nakai, which does not sprout flower stalks in Fall can be used as a medicine. In this regard, the roots of the female herb are cleaned with water and dried under sunshine. On the other hand, the roots of male *Angelica gigas Nakai* are not used as a medicine. Coumarin is obtained at a yield of 1.38 % from the leaves and at a yield of 2-3 % from the roots. The roots also contain pyrano-coumarin such as decursin C₁₉H₂₀O₅, decrusinol C₁₄H₁₄O₄, nodakenetin C₁₄H₁₄O₄, umbelliferon, and nodakenin. In addition, other coumain derivatives such as xanthotoxin, yasopimpineline, ostol, and umbellifrenin are isolated from the roots. Further, roots have essential oils (0.3-0.6 %) and resinous materials. Decursinol is found in fruits of the herb. Like the roots, the leaves contain essential oils and coumain in an amount of 1 %. The essential oils and coumain derivatives of the roots are found to have a variety of medicinal functions, including sedation, contraparetion, blood pressure depression, and pain relief and anti-inflammation for arthritis. In Oriental medicine, the roots are used as a hematic, an anodyne, a sedative, and a cordial for the treatment of anemia and obstetric/gynecological diseases. In combination with other medicinal herbs, the roots are particularly prescribed for women who suffer from irregular menstruation, menstrual colic, and uterine hypoplasia, and who are staying for the benefit of their health at home or hospitals after parturition.

Astragalus *membranaceus* (*Fisch*.) *Bunge* is a perennial herb with a height of 1-1.5 m. From the roots, 2',4'-dihydroxy-5',6'-dimethoxyisoflavan, 5,4'-dioxy-3,7-dimethoxyflavon, a flavonoid (C₁₆H₁₂O₅), and coumatagenin are isolated. The roots also contain β-sitosterol, fructose, glucose, starch, mucous materials, alkaloids, saponin-reactive materials, choline, betain, linolic acid, linoleic acid, and amino acids such as leucine (16 mg%), glycine (24 mg%), serine (28 mg%), alanine (55 mg%), glutamic acid (77 mg%), arginine (83 mg%), and γ-aminobutyric acid (24-66 mg%). With medicinal functions, including sedation, blood pressure depression, and vasodilation, *Astragalus membranaceus (Fisch.) Bunge* is used for the treatment of hypertension, cardiovascular dysfunction, and acute and chronic heart diseases. In Oriental medicine, this herb is used as a cordial, a restorative, and a diuretic, so that it is applied for promoting the function of the kidneys and invigorating the body.

Roots of *Paeonia aliflora Pallas var. trichocarpa Bunge* are used as a medicinal material after being peeled and dried under sunshine. Glycosides such as paeonolide and paeoniflorin C₂₂H₂₈O₁₁ are obtained in the amount of 1.5-6 %, along with paeonin, tannin materials, resinous materials, sugars, starch, salicylic acid (0.37 %), methylsalicylate, and essential oils. Water or alcohol extracts from the roots show sedation and analgesic functions and increase the acidity of the gastric juice. They are also found to stimulate intestinal peristalsis and have inhibitory activity against some pathogenic bacteria. The alkaloid paeonin shows medicinal effects beneficial to the womb in addition to having functions similar to those of the alkaloid aconitine. Paeoniflorin shows useful medicinal functions, including sedation, weak antiphlogistic function, and ulcer-preventive function, without affecting respiration. In addition, the herb is found to have effects of antihypertension, sedation, pain alleviation, anti-inflammation, stress ulcer-preventive function, relaxation of smooth muscle, and vasodilation. In Oriental medicine, the herb is used as an anodyne and a sedative for the treatment of longissimus dorsi convulsion, neuralgia, menstrual colic, and abdominal ache.

*Atractylodes japonica (Koidz.) Kitag* is a perennial herb about 80 cm high. In Oriental medicine, its root is useful. For use in a medicinal material, roots of Atractylodes japonica (Koidz) Kitag are collected in Spring or Fall and deprived of rootlets, followed by drying under sunshine. Dried, thick and massive roots which are stripped of their skin are known to show more potent medicinal effects. The roots contain essential oils in the amount of about 1.5 % by weight as well as carotin, inulin, and alkaloid. The essential oils extracted from the root are found to have the functions of sedation, central nerve paralysis, hypotension, and promotion of urination, in addition to being used as an aromatic stomachic for the treatment of dyspepsia. In Oriental medicine, the root of *Atractylodes japonica (Koidz.) Kitag* is described to promote excretion of water from the body. Accordingly, it is used as a diuretic for the treatment of renal failure, and as a hidrotic. It is also prescribed for the patients who suffer from diarrhea, nausea, diabetes mellitus, pulmonary tuberculosis, coughing, rheumatoid arthritis, gout, fever, cold, hepatic diseases, splenopathy, and malignant tumors.

Fruits of *Crataegus pinnatifida Bunge* contain amygdalin, organic acids such as ursolic acid (C₃₀H₄₃O₃), chlorogenic acid, lemon acid, and wine acid, flavonoids, and vitamin C. In addition, many other components remain unidentified. Alcohol extracts from the fruits are found to reduce the excitation of the cerebral nerves, as well as exerting continuous sedation effects. With a cordial effect, the fruit extract considerably improves the activity of the heart and shows antagonistic activity against the dysrhythmia caused by aconitine. In Oriental medicine, the fruit is used as a digestive, an antidiuretic, and a sedative, and is prescribed for patients afflicted with dyspepsia or stomach upset. Also, its medications are applied for leukemia, pain, and disorders of the biliary tract.

Fruits or fruit rinds of *Zanthoxylum piperitum (L.) DC.* contain essential oils in the amount of 2 to 4 % by weight, which consist mainly of dipentene (C₁₀H₁₆, 54 %), citronellal (C₁₀H₁₈, 8 %), 1-β- phellandrene (C₁₀H₁₆), geraniol, and citronellol. Additionally, the characteristic spicy taste of the fruits is attributed to zanthol I and II that they contain in the amounts of 5 to 8 %, respectively. The components are known to exhibit local anesthetic effects. The fruit shows more potent anesthetic effects as it becomes more mature. In addition, the fruit is found to have inhibitory activity against various pathogenic bacteria. In Oriental medicine, the fruit is used as an aromatic stomachic to treat gastroenteritis, gastric dilation, and gastroptosis. Also, it is applied as a diuretic, a local stimulant, and a vermicide.

Fruits of *Lycium chinense Miller* contain vitamins and zeaxanthine. Recently, scopletin, a biologically active material, has been isolated from the fruits. Also, there were isolated carotinoid and sterin, which are identified as physalien and β-sitosterin, respectively. In Oriental medicine, the fruits of *Lycium chinense Miller* are utilized as a hypotensive agent. Found to have the function of lowering blood cholesterol, the fruits are used for the prophylaxis and treatment of arteriosclerosis. In some historical medical books, the fruits are described to be useful for the treatment of lumbago, asthenia, vertigo, headache, and diabetes mellitus. It is also described that humans who have been administered with the fruit for a long period of time may enjoy medicinal effects including increased bone density, vigor, keen eyesight, resistance to cold and heat, and longevity.

Fruits of *Macrocarpium officinale Sieb. et Zucc.* contain crystalline organic acids, gallic acid, malic acid, tartaric acid, etc. In the skin of the fruits are found morroniside, loganin and sworoside. In Oriental medicine, the fruits are used to aid renal function and applied to persons who often sweat or urinate in small amounts, or suffer from lumbago or irregular menstruation.

*Glycyrrhiza glabra L.* is a perennial herb which grows to a height of 30-80 cm. Its roots and root stems from which glycyrrhizin is isolated in the amount of 5-23 %, along with glycyrrhetinic acid (C30H46O5) are medicinally useful. Glycyrrhizin, known as a medicinally useful compound, is 40-50 fold sweeter than sugar and promotes the function of the respiratory tract. Glycyrrhetinic acid, generated by the hydrolysis of glycyrrhizin, lacks the sweet taste and has hemolyzation. Some research has revealed that the root of *Glycyrrhiza glabra L.* has expectorant, anti-inflammation, sedation, and anti-histamine activity. In Oriental medicine, the root is used to sweeten tastes of decoctions of almost all herbal medicines and neutralize the toxins that the medicinal herbs contain. Recently, it has been applied for the treatment of gastric and duodenal ulcer, Adison's disease, bronchial asthma, jaundice, hepatitis, and eczema.

As described above, the materials used in the present invention are obtained from herbs which grow naturally and are safe to the body. For use in the composition of health foods for the prophylaxis and treatment of constipation, the medicinal herbs, *Angelica gigas Nakai*, Astragalus *membranaceus (Fisch.) Bunge,* roots of *Paeonia aliflora Pallas var. trichocarpa Bunge*, *Atractylodes japonica (Koidz.) Kitag*, fruits of *Crataegus pinnatifida Bunge*, fruits or fruit rinds of *Zanthoxylum piperitum (L.) DC.,* fruits of *Lycium chinense Miller,* fruits of *Macrocarpium officinale Sieb. et Zucc.,* and *Glycyrrhiza glabra L.,* are dried and powdered. The powder mixture may be formulated in forms of tablets, granules or capsules, or used as materials of drinks or soups. Alternatively, the herbs may be extracted with hot water or organic solvents.

A better understanding of the present invention may be obtained in light of the following examples which are set forth to illustrate, but are not to be construed to limit the present invention.

### EXAMPLE 1

### Composition of Health Food for the Prophylaxis and Treatment of Constipation

A composition of a health food for the prophylaxis and treatment of constipation was prepared from the following components:

| | |
|---|---|
| *Angelica gigas Nakai* | 12wt% |
| *Astragalus membranaceus* (*Fisch*.) *Bunge* | 12wt% |
| Root of *Paeonia aliflora Pallas var. trichocarpa Bunge* | 12wt% |
| *Atractylodes japonica* (*Koidz*.) *Kitag* | 12wt% |
| Fruits of *Crataegus pinnatifida Bunge* | 12wt% |
| Fruits of *Zanthoxylum piperitum (L.) DC.* | 12wt% |
| Fruits of *Lycium chinense Miller* | 12wt% |
| Fruits of *Macrocarpium officinale Sieb. et Zucc.* | 12wt% |
| *Glycyrrhiza glabra* L. | 4wt% |

In the following animal tests, the composition was assayed for anti-constipation effect.

### EXPERIMENTAL EXAMPLE 1

### Effect of the Composition of Health Food on Constipation (Animal Test)

For assaying the composition of the present invention for anti-constipation activity, rats were quantitatively monitored for their stools and water contents thereof.

Before testing, 20 Sprague-Dawley female rats which were three weeks old were adapted to a new environment while being fed with pellet diets. They were divided into two groups of 10, which were raised in metabolic cages, separately. The composition was suspended in saline and injected abdominally at a dose of 0.14 mg per 250 g of body weight once a day for four weeks to the test group, while the other control group was administered with saline. During the time period of the injections, the rats were quantitatively monitored for stools and water content thereof at the same time (8:30 AM) every day. The results are given in Tables 1 and 2, below.

As seen in Tables 1 and 2, the test group to which the composition of health foods for the prophylaxis and treatment of constipation was injected had increased amounts of stools compared to the saline-administered control, and the composition is found to be useful for the treatment of constipation as demonstrated by the increase in the water content of stools.

**TABLE 1**

| Amount of Stools According to Administration Time Period | | | | | | | |
|---|---|---|---|---|---|---|---|
| Group | Day 16 | Day 18 | Day 20 | Day 22 | Day 24 | Day 26 | Day 28 |
| Control | 100 | 86.4 | 89.1 | 98.6 | 90.5 | 85.4 | 80.6 |
| Test | 100 | 138.1 | 150.5 | 164.7 | 153.4 | 142.1 | 138.3 |

**TABLE 2**

| Water Content of Stools According to Administration Time Period | | | | | |
|---|---|---|---|---|---|
| Group | Day 16 | Day 18 | Day 20 | Day 23 | Day 28 |
| Control | 100 | 93.2 | 95.4 | 90.7 | 89.1 |
| Test | 100 | 108.4 | 122.1 | 112.6 | 99.3 |

### EXPERIMENTAL EXAMPLE 2

### Effect of the Composition of Health Food on Constipation (Human Test)

15 men and 15 women, all in their twenties or thirties, who had been afflicted with constipation, were let to drink a beverage made of the composition of the present invention and interviewed for the change in their passage of stools. The interview results are given in Table 4, which were evaluated according to the criteria suggested in Table 3. As seen in Table 4, the subjects replied easy passage of stools with no feelings of residual stools. Also, they had had bowel movements once every other or three days before the testing, but were measured to have bowel movements once every day or every other day after the drinking of the composition. Therefore, the composition of the present invention is effective for the treatment of constipation as demonstrated by the human test.

**TABLE 3**

| Criteria | | |
|---|---|---|
| Item | Criteria | Decision of Improvement |
| Defecating Feeling | 1. Very strong urge, but difficult to defecate. | Subject tending toward criterion 3 |
| | 2. Small defecation with feelings of residual stools | |
| | 3. No difficulty in passing stools | |
| Frequency of Bowel Movements | 1. Once a day | Subject tending toward criterion 1 |
| | 2. Once every two days | |
| | 3. Once every three days | |
| | 4. Irregular | |
| Effect Grade | 0 to 5 | Answer for improvement from no effect (0) to excellent effect (5) of subject |

**TABLE 4**

| Result of Questionaire the Effect of the Composition on Anti-Constipation | | | | | | | | | | | | | |
|---|---|---|---|---|---|---|---|---|---|---|---|---|---|
| | Defecating Feeling | | | Freq. of bowel movements | | | | Effect Grade | | | | | |
| | 1 | 2 | 3 | 1 | 2 | 3 | 4 | 0 | 1 | 2 | 3 | 4 | 5 |
| Before Drinking | 14 | 16 | 0 | 0 | 8 | 9 | 13 | | | | | | |
| After Drinking | 8 | 11 | 11 | 11 | 16 | 5 | 5 | 1 | 1 | 3 | 5 | 4 | 16 |

As described hereinbefore, a composition of health food is provided for preventing and treating constipation in accordance with the present invention. Made of medicinal herbs, the composition is safe to the liver and exhibits excellent pharmaceutical effects and provide comfort for the user without side effects.

The present invention has been described in an illustrative manner, and it is to be understood that the terminology used is intended to be in the nature of description rather than of limitation. Many modifications and variations of the present invention are possible in light of the above teachings. Therefore, it is to be understood that within the scope of the appended claims, the invention may be practiced otherwise than as specifically described.

## Claims

1. A composition of health foods for the prophylaxis and treatment of constipation, comprising:
8-15 % by weight of *Angelica gigas Nakai*,
8-15 % by weight of Astragalus membranaceus (Fisch.) Bunge,
8-15 % by weight of Paeonia aliflora Pallas var. trichocarpa Bunge,
8-15 % by weight of Atractylodes japonica (Koidz.) Kitag,
8-15 % by weight of Crataegus pinnatifida Bunge,
8-15 % by weight of Zanthoxylum piperitum (L.) DC.,
8-15 % by weight of *Lycium* chinense *Miller,*
8-15 % by weight of Macrocarpium officinale Sieb. et Zucc., and
2-20 % by weight of *Glycyrrhiza glabra L.*
